## Europäisches Patentamt

(19) **European Patent Office**

**Office européen des brevets**

(11) Veröffentlichungsnummer: **0 017 851**
**B1**

(12) **EUROPÄISCHE PATENTSCHRIFT**

(45) Veröffentlichungstag der Patentschrift:
**04.11.81**

(21) Anmeldenummer: **80101750.0**

(22) Anmeldetag: **02.04.80**

(51) Int. Cl.³: **C 07 C 39/11, C 07 C 37/20**

(54) Verfahren zur Herstellung eines Gemisches aus 2- und 4-Hydroxybenzylalkohol.

(30) Priorität: **14.04.79 DE 2915216**

(43) Veröffentlichungstag der Anmeldung:
**29.10.80 Patentblatt 80/22**

(45) Bekanntmachung des Hinweises auf die Patenterteilung:
**04.11.81 Patentblatt 81/44**

(84) Benannte Vertragsstaaten:
**BE CH DE FR GB IT LI NL**

(56) Entgegenhaltungen:
**DE-A1-2 545 338**
**DE-C-85 586**

(73) Patentinhaber: **HAARMANN & REIMER GMBH,**
**Postfach 138, D-3450 Holzminden (DE)**

(72) Erfinder: **Bauer, Kurt, Dr., Corveyblick 41,**
**D-3450 Holzminden (DE)**
Erfinder: **Mölleken, Reiner, Dr., Bergstrasse 8,**
**D-3450 Holzminden (DE)**
Erfinder: **Wedemeyer, Karlfried, Dr., Bilharzstrasse 7,**
**D-5000 Köln 80 (DE)**
Erfinder: **Fiege, Helmut, Dr., Walter-Flex-Strasse 8,**
**D-5090 Leverkusen 1 (DE)**

(74) Vertreter: **Dill, Erwin, Dr. et al, c/o BAYER AG**
**Zentralbereich Patente Marken und Lizenzen Bayerwerk,**
**D-5090 Leverkusen 1 (DE)**

# 0 017 851

## Verfahren zur Herstellung eines Gemisches aus 2- und 4-Hydroxybenzylalkohol

Die Erfindung betrifft ein Verfahren zur Herstellung eines Gemisches aus 2- und 4-Hydroxybenzylalkohol.

In der DE-OS 27 29 075 ist ein Verfahren zur Herstellung von 2- und 4-Hydroxybenzylalkohol-Gemischen beschrieben. Nach diesem Verfahren werden die Gemische in hohen Raum/Zeit-Ausbeuten durch Umsetzung von Phenol und Paraformaldehyd in Gegenwart stark basischer Katalysatoren erhalten. In diesen Gemischen beträgt jedoch der Anteil des 4-Hydroxybenzylalkohols nur etwa 30 Gew.-%, bezogen auf die im Reaktionsgemisch vorhandenen Hydroxybenzylalkohole.

Da 4-Hydroxybenzylalkohol ein wichtiges Ausgangsmaterial für die Herstellung von 4-Hydroxybenzaldehyd darstellt, bestand das Bedürfnis, Gemische von 2- und 4-Hydroxybenzylalkohol mit einem höheren Anteil an 4-Hydroxybenzylalkohol herzustellen.

Es wurde überraschenderweise gefunden, daß sich der Anteil an 4-Hydroxybenzylalkohol in besagten Hydroxybenzylalkohol-Gemischen wesentlich erhöhen läßt, wenn man die Umsetzung von Phenol und Paraformaldehyd unter Verwendung stark basischer Katalysatoren in Gegenwart von Polyalkylenpolyethern durchführt.

Die Erfindung betrifft daher ein Verfahren zur Herstellung von Gemischen aus 2- und 4-Hydroxybenzylalkohol durch Umsetzung von Phenol und Paraformaldehyd in Gegenwart stark basischer Katalysatoren, das dadurch gekennzeichnet ist, daß man die Umsetzung in Gegenwart von Polyalkylenpolyethern durchführt.

Die Polyalkylenpolyether weisen vorteilhaft ein Molekulargewicht von etwa 140 bis 14 000 auf. Sie können offenkettige oder cyclische Ether sein.

Als offenkettige Polyalkylenpolyether seien beispielsweise genannt:

Polyalkylenglykole und deren Mono- und Di-ether, z. B. Polyethylenglykole, Polypropylenglykole, gemischte Polyethylen-Propylen-Glykole und deren Alkyl-, Aralkyl- und Arylether, z. B. Methyl-, Ethyl-, Propyl-, Butyl-, Pentyl-, Hexyl-, Benzyl-, Phenyl-, $C_1$–$C_{12}$-Alkyl-phenylether.

Als Vertreter seien beispielsweise genannte Polyethylenglykole der Molekulargewichte 1500, 3600 und 12 000, Triethylenglykolmonoethylether, Polyethylenglykoldimethylether, Tetraethylenglykolmonoethylether; Polypropylenglykole der Molekulargewichte 400–5000; mit 16 bis 24 Mol Ethylenoxid je Mol Phenol umgesetztes Octyl-, Nonyl- oder Dodecylphenol.

Als cyclische Polyether seien beispielsweise die als »Kronenether« bezeichneten mittel- bis vielgliedrigen cyclischen Polyether genannt, in denen die Ether-Sauerstoffatome durch Alkylenvorzugsweise Ethylenbrücken verbunden sind und die ein oder mehrere ankondensierte Benzol-, Cyclohexan- oder heteroaromatische Ringe enthalten können (s. J. Am. Chem. Soc. 89, 7017 (1967); Chem. Rev. 74, 354 (1974); Chem. Commun. 1976, 295). Als Vertreter der Kronenether seien beispielsweise genannt:

1,4,7,10-Tetraoxacyclododecan (12-Krone-4),
1,4,7,10,13-Pentaoxacyclopentadecan (15-Krone-5),
1,4,7,10,13,16-Hexaoxacyclooctadecan (18-Krone-6),
2,5,8,15,18,21-Hexaoxatricyclo[20,4,0,0,9,14]hexacosan (Dicyclohexyl-18-Krone-6),
1,4,7,14,23-Pentaoxa[7,2]orthocyclo[2](2,6)-pyridinophan (Dibenzopyridino-18-Krone-6),
1,13-Bis-(8-chinolyl)-1,4,7,10,13-pentaoxatridecan.

Die erfindungsgemäß zu verwendenden Polyalkylenpolyether werden in einer Menge von 0,05 bis 10 g, vorzugsweise 0,1 bis 5 g je Mol Phenol eingesetzt.

Als stark basische Katalysatoren werden in dem erfindungsgemäßen Verfahren vorzugsweise Natrium- oder Kaliumhydroxid verwendet. Die Katalysatoren werden in einer Menge von 0,0001 bis 0,05 Mol, vorzugsweise 0,0005 bis 0,02 Mol je Mol Phenol eingesetzt.

In dem erfindungsgemäßen Verfahren werden Phenol und Paraformaldehyd im Molverhältnis 5–15 : 1, vorzugsweise 8–12 : 1, eingesetzt.

Polyalkylenpolyether und stark basischer Katalysator können den Reaktionskomponenten zu Reaktionsbeginn zugesetzt werden. In manchen Fällen hat es sich jedoch als vorteilhaft erwiesen, den stark basischen Katalysator nicht wie die Polyalkylenpolyether mit einem Male zu Reaktionsbeginn zuzusetzen, sondern zu Beginn der Reaktion nur etwa ein Drittel der Gesamtkatalysatormenge und die restlichen zwei Drittel im Verlauf der Reaktion zuzugeben.

Das erfindungsgemäße Verfahren wird vorteilhaft bei Temperaturen von 25 bis 120° C, vorzugsweise 40 bis 90° C, ausgeführt. Die Reaktionszeit schwankt zwischen 0,5 und 6 Stunden.

Die Aufarbeitung des Reaktionsgemisches kann, wie in der DE-OS 27 29 075 beschrieben durch teilweises Abdestillieren des überschüssigen Phenols und Isolierung der 2- und 4-Hydroxybenzylalkohole durch Gegenstromextraktion erfolgen.

## Beispiel 1

1,25 Mol Phenol und 0,125 Mol Paraformaldehyd (Wassergehalt: 3,6 Gew.-%) werden unter Rühren auf 65° C erwärmt. Nach Zugabe von 0,08 g 50%igem wäßrigem Kaliumhydroxids (0,0007 Mol) und 0,75 g Polyethylenglykol (Molekulargewicht 400) wird die Reaktionsmischung 4 Stunden auf 65°C gehalten. Dann werden weitere 0,16 g 50%iges wäßriges Kaliumhydroxid (0,0014 Mol) zugegeben und die Umsetzung durch 2stündiges Rühren bei 65°C zuende geführt.

Anschließend wird das Kaliumhydroxid mit der äquivalenten Menge Essigsäure neutralisiert und ein Teil des überschüssigen Phenols zunächst im Fallfilmverdampfer, danach im Dünnschichtverdampfer bei 55 bis 60°C und 3 bis 5 Torr abdestilliert.

In dem so erhaltenen Reaktionsgemisch werden die Anteile von 2- und 4-Hydroxybenzylalkohol durch Hochdruck-Flüssigkeitschromatographie bzw. — nach Acetylieren des Reakionsgemisches — durch Gaschromatographie bestimmt. Das Reaktionsgemisch enthält 53,4% 2-Hydroxybenzylalkohol und 46,6% 4-Hydroxybenzylalkohol (bezogen auf den Gesamtanteil an Hydroxybenzylalkoholen im Reaktionsgemisch).

## Beispiele 2 bis 13

1,25 g Phenol und 0,125 Mol Paraformaldehyd (Wassergehalt: 3,6 Gew.-%) wurden bei 65°C unter Verwendung der in Tabelle 1 angegebenen Menge 50%igen wäßrigen Kaliumhydroxids in Gegenwart der in Tabelle 1 angegebenen Polyalkylenpolyether 6 Stunden in der im Beispiel 1 beschriebenen Weise umgesetzt.

Die Analyse der bei diesen Umsetzungen angefallenen Hydroxybenzylalkohol-Gemische ergab die in Tabelle 1 angegebenen Gehalte an 2- und 4-Hydroxybenzylalkohol (alle bezogen auf den Gesamthydroxybenzylalkohol-Gehalt der Reaktionsgemische).

Tabelle 1

| Beispiel | Phenol [Mol] | KOH (50%) [Mol] | [g] | Polyalkylenpolyether [g] | 2-Hydroxyben- zylalkohol [%] | 4-Hydroxy- benzylalkohol [%] |
|---|---|---|---|---|---|---|
| 2 | 1,25 | 0,002 | 0,22 | Polyäthylenglykol (MG 1500) 0,75 | 56,8 | 43,2 |
| 3 | 1,25 | 0,002 | 0,22 | Polyäthylenglykol (MG12 000) 0,75 | 57,0 | 43,0 |
| 4 | 1,25 | 0,002 | 0,22 | Polypropylenglykol (MG 3600) 0,75 | 60,7 | 39,3 |
| 5 | 1,25 | 0,002 | 0,22 | Polyäthylenglykol- monononylphenylether (MG 2100) 0,75 | 58,8 | 41,2 |
| 6 | 1,25 | 0,002 | 0,22 | Triäthylenglykol- monomethylether 0,75 | 57,6 | 42,4 |
| 7 | 1,25 | 0,002 | 0,22 | Polyäthylenglykol- dimethylether (MG 200) 0,75 | 55,3 | 44,7 |
| 8 | 5 | 0,003 | 0,34 | Polyäthylenglykol- dimethylether (MG 200) 2,0 | 59,9 | 40,1 |
| 9 | 5 | 0,003 | 0,34 | Polyäthylenglykol- dimethylether (MG 200) 5,0 | 55,1 | 44,9 |
| 10 | 1,25 | 0,002 | 0,22 | [12] Krone-4-Ether 0,75 | 56,3 | 43,7 |
| 11 | 1,25 | 0,002 | 0,22 | [15] Krone-5-Ether 0,75 | 53,3 | 46,7 |
| 12 | 5 | 0,008 | 0,9 | [18] Krone-6-Ether 1,3 | 51,0 | 49,0 |
| 13 | 5 | 0,008 | 0,9 | [18] Krone-6-Ether 1,13 | 53,1 | 46,9 |

Beispiele 14 bis 20

1,25 Mol Phenol und 0,125 Mol Paraformaldehyd (Wassergehalt: 3,6 Gew.-%) wurden bei 65°C unter Verwendung des in Tabelle 2 angegebenen stark basischen Katalysators in Gegenwart der in Tabelle 2 angegebenen Polyalkylenpolyether 6 Stunden in der in Beispiel 1 beschriebenen Weise umgesetzt, mit der Abänderung, daß die gesamte Katalysatormenge zu Beginn der Reaktion zugesetzt wurde.

Die Analyse der bei diesen Umsetzungen anfallenden Hydroxybenzylalkohol-Gemische ergab die in Tabelle 2 angegebenen Gehalte an 2- und 4-Hydroxybenzylalkohol (bezogen auf den Gesamthydroxy-benzylalkohol-Gehalt im Reaktionsgemisch).

Tabelle 2

| Beispiel | Phenol [Mol] | Katalysator [Mol] [g] | Polyalkylenpolyether [g] | 2-Hydroxyben-zylalkohol [%] | 4-Hydroxy-benzylalkohol [%] |
|---|---|---|---|---|---|
| 14 | 5 | KOH (50%) 0,008 0,9 | Polyäthylenglykol-dimethylether (MG 200) 5 | 58,2 | 41,8 |
| 15 | 5 0,02 | NaOH 0,76 4 | [15] Krone-5-Ether | 54,8 | 45,2 |
| 16 | 5 | KOH (50%) 0,008 0,9 | [18] Krone-6-Ether 1,0 | 62,0 | 38,0 |
| 17 | 5 | KOH (50%) 0,008 0,9 | [18] Krone-6-Ether 1,3 | 62,0 | 38,0 |
| 18 | 5 | KOH (50%) 0,0035 0,4 | Dibenzo-[18] Krone-6-Ether 1,8 | 52,8 | 47,2 |
| 19 | 5 | KOH (50%) 0,0035 0,4 | Dicyclohexyl-[18] Krone-6-Ether 1,8 | 54,1 | 45,9 |
| 20 | 5 | KOH (50%) 0,0035 0,4 | Dibenzopyridino-[18] Krone-6-Ether 1,9 | 54,4 | 45,6 |
| Ver-gleichs-beispiel | 5 | KOH (50%) 0,01 1,2 | – | 70,0 | 30,0 |

## Patentansprüche

1. Verfahren zur Herstellung eines Gemisches aus 2- und 4-Hydroxybenzylalkohols durch Umsetzung von Phenol und Paraformaldehyd in Gegenwart stark basischer Katalysatoren, dadurch gekennzeichnet, daß man die Umsetzung in Gegenwart von Polyalkylenpolyethern vornimmt.

2. Verfahren nach Anspruch 1, dadurch gekennzeichnet, daß die Polyalkylenpolyether ein Molekulargewicht von 140 bis 14 000 aufweisen.

3. Verfahren nach Anspruch 1 und 2, dadurch gekennzeichnet, daß man als Polyalkylenpolyether Polyalkylenglykole, deren Mono- oder Diether und/oder Kronenether verwendet.

4. Verfahren nach Anspruch 1 bis 3, dadurch gekennzeichnet, daß man als Polyalkylenpolyether Polyethylenglykole und/oder deren Mono- oder Diether verwendet.

## Claims

1. Process for the preparation of a mixture of 2- and 4-hydroxybenzyl alcohol by reaction of phenol and paraformaldehyde in the presence of strongly basic catalysts, characterised in that the reaction is carried out in the presence of polyalkylene polyethers.

2. Process according to claim 1, characterised in that the polyalkylene polyethers have a molecular weight of 140 to 14 000.

3. Process according to claims 1 and 2, characterised in that polyalkylene glycols, monoethers or diethers thereof and/or crown ethers are used as the polyalkylene polyethers.

4. Process according to claims 1 to 3, characterised in that polyethylene glycols and/or monoethers or diethers thereof are used as the polyalkylene polyethers.

## 0 017 851

**Revendications**

1. Procédé de préparation d'un mélange d'alcools 2- et 4-hydroxybenzyliques par réaction du phénol et du paraformaldéhyde en présence de catalyseurs fortement basiques, caractérisé en ce que l'on effecture la réaction en présence de polyalkylènepolyéthers.

2. Procédé selon la revendication 1, caractérisé en ce que les polyalkylène-polyéthers présentent un poids moléculaire de 140 à 14 000.

3. Procédé selon les revendications 1 et 2, caractérisé en ce que l'on utilise en tant que polyalkylène-polyéthers des polyalkylèneglycols, leurs mono- ou di-éthers et/ou des éthers en couronne.

4. Procédé selon les revendications 1 à 3, caractérisé en ce que l'on utilise en tant que polyalkylène-polyéthers des polyéthylène-glycols et/ou leurs mono- et di-éthers.